# EUROPÄISCHE PATENTANMELDUNG

(11) **EP 0 714 904 A1**
(43) Veröffentlichungstag der Anmeldung: **05.06.1996**
(21) Anmeldenummer: 95118258.3
(22) Anmeldetag: 21.11.1995
(51) Int. Cl.: C07H 13/04, C09K 19/34

(54) **Chirale Verbindungen**

(30) Priorität: 30.11.1994 DE 44426143
(71) Anmelder: BASF AKTIENGESELLSCHAFT, D-67056 Ludwigshafen (DE)
(72) Erfinder: Siemensmeyer, Karl, Dr., D-67227 Frankenthal (DE); Vill, Volkmar, Dr., D-48145 Münster (DE); Tunger, Hanns-Walter, D-22085 Hamburg (DE); Paul, Matthias, D-22589 Hamburg (DE)

(57) **Zusammenfassung**

Chirale Verbindungen der allgemeinen Formel I 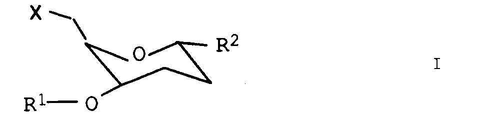
in der
- R¹: Wasserstoff oder ein Rest der Formel -Y-Z-Y¹,
- R²: ein Rest der Formel

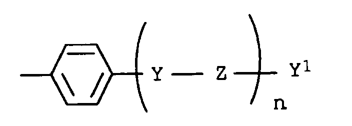

und
- X: Wasserstoff, Fluor, Chlor, Brom, Jod, Hydroxy oder OR¹ sind, wobei
- Y: eine direkte Bindung, CO oder C₂- bis C₈-Alkylen, in welchem nichtbenachbarte C-Atome durch O, CO, COO oder OCO ersetzt sein können
- Z: eine Gruppe, die einen oder mehrere gesättigte oder ungesättigte, gegebenenfalls Heteroatome aufweisende Ringe enthält,
- Y¹: C₁- bis C₁₅-Alkyl, C₂- bis C₁₅-Alkenyl, C₁- bis C₁₅-Alkoxy, C₂- bis C₁₅-Alkenyloxy, C₁- bis C₁₅-Alkanoyloxy oder C₃- bis C₁₅-Alkenoyloxy, wobei in diesen Resten nichtbenachbarte Kohlenstoffatome durch Sauerstoff, OCO oder COO ersetzt sein können, und
- n: 0,1 oder 2 bedeuten.

## Beschreibung

Wie für formanisotrope Medien bekannt, können beim Erwärmen flüssigkristalline Phasen, sogenannte Mesophasen, auftreten. Die einzelnen Phasen unterscheiden sich durch die räumliche Anordnung der Molekülschwerpunkte einerseits sowie durch die Molekülanordnung hinsichtlich der Längsachsen andererseits (G.W. Gray, P.A. Winsor, Liquid Crystals and Plastic Crystals, Ellis Horwood Limited, Chichester 1974). Die nematisch flüssigkristalline Phase zeichnet sich dadurch aus, daß lediglich eine Orientierungsfernordnung durch Parallellagerung der Moleküllängsachsen existiert. Unter der Voraussetzung, daß die die nematische Phase aufbauenden Moleküle chiral sind, entsteht eine sogenannte cholesterische Phase, bei der die Längsachsen der Moleküle eine zu ihnen senkrechte, helixartige Überstruktur ausbilden (H. Baessler, Festkörperprobleme XI, 1971). Der chirale Molekülteil kann im flüssigkristallinen Molekül selbst vorhanden sein oder als Dotierstoff zur nematischen Phase gegeben werden, wodurch die cholesterische Phase induziert wird. Dieses Phänomen wurde zuerst mit Cholesterolderivaten untersucht (z.B. H. Baessler, M.M. Labes, J.Chem.Phys., 52,631 (1970); H.Baessler, T.M. Laronge, M.M. Labes, J.Chem.Phs., 51,799 (169); H.Finkelmann, H.Stegemeyer, Z.Naturforschg. 28a,799 (1973); H.Stegemeyer, K.J. Mainusch, Naturwiss., 58,599 (1971), H.Finkelmann, H.Stegemeyer, Ber.Bunsenges.Phys.Chem. 78,869 (1974)).

Die cholesterische Phase besitzt bemerkenswerte optische Eigenschaften: eine hohe optische Rotation sowie einen ausgeprägten Zirkulardichroismus, der durch Selektivreflektion von zirkular polarisiertem Licht innerhalb der cholesterischen Schicht entsteht. Die je nach Blickwinkel zu beobachtenden unterschiedlichen Farben sind abhängig von der Ganghöhe der helixartigen Überstruktur, die ihrerseits vom Verdrillungsvermögen der chiralen Komponente abhängt. Dabei kann insbesondere durch Änderung der Konzentration eines chiralen Dotierstoffes die Ganghöhe und damit der Wellenlängenbereich des selektiv reflektierten Lichtes einer cholesterischen Schicht variiert werden. Solche cholesterischen Systeme bieten für eine praktische Anwendung interessante Möglichkeiten. So kann durch Einbau chiraler Molekülteile in mesogene Acrylsäureester nach Einstellung der cholesterischen Orientierung und Photovernetzung ein stabiles, farbiges Netzwerk hergestellt werden, dessen Konzentration an chiraler Komponente aber nicht verändert werden kann (G.Galli, M.Laus, A.Angelon, Makromol. Chemie. 187,289 (1986)). Ebenso kann durch Zumischen von nichtvernetzbaren chiralen Verbindungen zu nematischen Acrylsäureestern nach Photovernetzung ein farbiges Polymer hergestellt werden, welches noch hohe Anteile löslicher Komponenten enthält (I. Heyndricks, D.J. Broer, Mol.Cryst.Liq. Cryst. 203,113 (1991)).

Auf ähnliche Weise kann durch statistische Hydrosilylierung von Cholesterolderivaten und acrylathaltigen Mesogenen mit definierten zyklischen Siloxanen nach Photopolymerisation ein cholesterisches Netzwerk hergestellt werden. Die chirale Komponente hat dabei einen Anteil von bis zu 50 % an den Ausgangsmaterialien, wodurch deutliche Mengen löslicher Anteile entstehen. (F.H. Kreuzer, R.Maurer, Ch.Müller-Rees, J. Stohrer, Vortrag Nr. 7, 22. Freiburger Arbeitstagung Flüssigkristalle, Freiburg, 1993). In der Anmeldung DE-OS 35 35 547 wird ein Verfahren beschrieben, bei dem eine Mischung cholesterinhaltiger Monoacrylate über eine Photovernetzung zu cholesterischen Schichten verarbeitet werden kann. Allerdings beträgt dabei der Gesamtanteil der chiralen Komponente in der Mischung ca. 94 %. Als reines Seitenkettenpolymer ist ein solches Material mechanisch nicht sehr stabil, die Stabilität kann jedoch durch hochvernetzende Verdünnungsmittel erreicht werden.

Neben den oben beschriebenen nematischen und cholesterischen Netzwerken sind auch smektische Netzwerke bekannt, welche insbesondere durch Photopolymerisation/Photovernetzung von smektisch flüssigkristallinen Materialien in der smektisch flüssigkristallinen Phase hergestellt werden. Die hierfür verwendeten Materialien sind in der Regel symmetrische, flüssigkristalline Bisacrylate wie sie z.B. in D.J. Broer, R.A.M. Hikmet, Makromol.Chem., 190,3201 (1989) beschrieben werden. Diese Verbindungen weisen aber sehr hohe Klärtemperaturen von >120°C auf, so daß die Gefahr einer thermischen Polymerisation gegeben ist. Durch Zumischung von nicht polymerisierbaren chiralen Materialien bei Vorliegen einer S_{C}*-Phase ist das Auftreten piezoelektrischer Eigenschaften im resultierenden Netzwerk möglich. (R.A.M. Hikmet, Macromolecules, 25,5759 (1992)).

Für die Anwendung chiraler Komponenten in LC-Mischungen ist es wünschenswert, möglichst hochverdrillende Verbindungen zur Verfügung zu haben, um den Anteil dieser Komponenten an der gesamten Mischung möglichst gering zu halten und weitere Eigenschaften nicht zu sehr zu verändern. Weiterhin sollen die chiralen Komponenten in der flüssigkristallinen Wirtsphase gut löslich sein.

Aufgabe der vorliegenden Erfindung war daher die Herstellung neuer chiraler Verbindungen, die hoch verdrillend sind und möglichst-um die Löslichkeit zu erhöhen-selbst eine flüssigkristalline Phase aufweisen .

Diese Aufgabe wird durch neue Verbindungen der allgemeinen Formel I 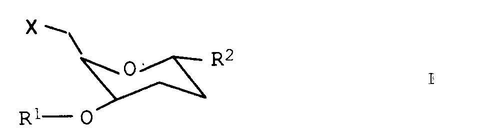
gelöst, in der
- R¹: Wasserstoff oder ein Rest der Formel -Y-Z-Y¹,
- R²: ein Rest der Formel 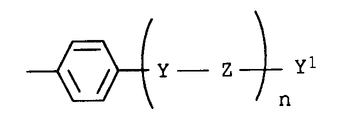
und
- X: Wasserstoff, Fluor, Chlor, Brom, Jod, Hydroxy oder OR¹ sind, wobei
- Y: eine direkte Bindung, CO oder gegebenenfalls durch O, COO oder OCO unterbrochenes C₂ bis C₈-Alkylen,
- Z: eine Gruppe, die einen oder mehrere gesättigte oder ungesättigte, gegebenenfalls Heteroatome aufweisende Ringe enthält,
- Y¹: C₁- bis C₁₅-Alkyl, C₂- bis C₁₅-Alkenyl, C₁- bis C₁₅-Alkoxy, C₂- bis C₁₅-Alkenyloxy, C₁- bis C₁₅-Alkanoyloxy oder C₃- bis C₁₅-Alkenoyloxy, wobei die Reste Y¹ durch Sauerstoff, OCO oder COO unterbrochen sein können, und
- n: 0,1 oder 2 bedeuten.

Reste Z sind z.B.:

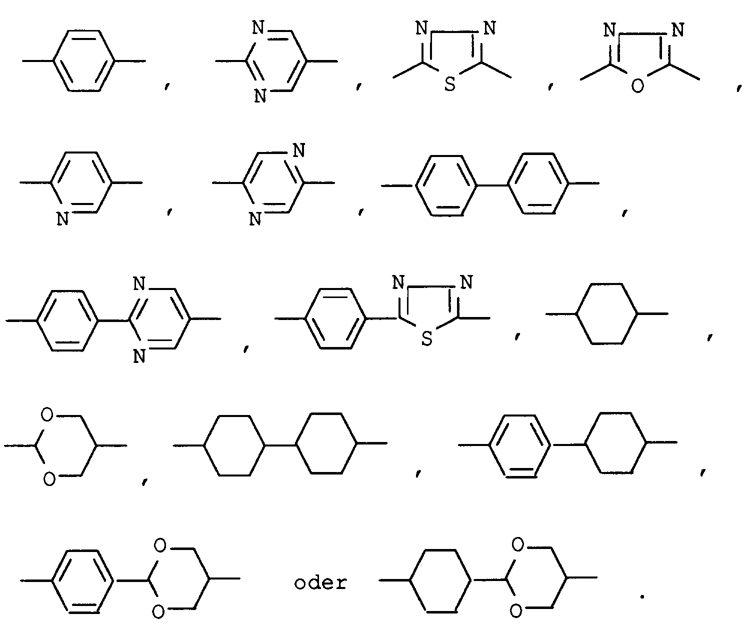

Von diesen sind 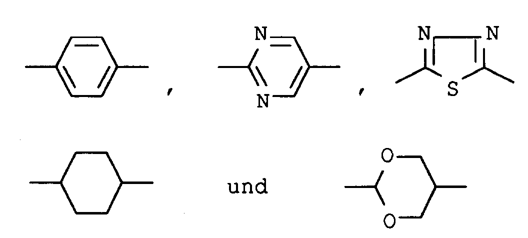
bevorzugt. Besonders bevorzugt ist der Rest

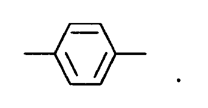

Als Brückenglieder Y kommen beispielsweise in Betracht:
-C₂H₄-, -C₃H₆-, -C₄H₈-, -C₅H₁₀-, -C₆H₁₂-, -C₇H₁₄-, -C₈H₁₆-,

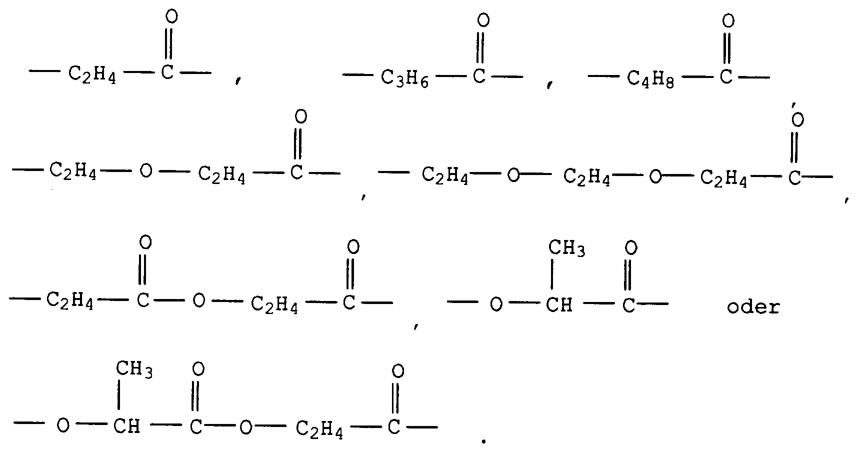

Bevorzugte Brückenglieder Y sind eine direkte Bindung, CO oder C₁-C₈-Alkylen, in welchem nicht benachbare C-Atome durch O, CO, COO oder OCO ersetzt sein können, darunter besonders bevorzugt: 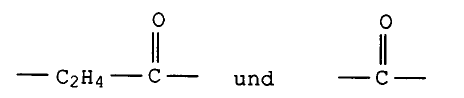
Y¹ ist bevorzugt C₁- bis C₁₅-Alkyl, C₁-C₁₅-Alkoxy, C₂-C₁₅-Alkenyl, C₂-C₁₅-Alkenyloxy, Acryloyloxy oder Methacryloyloxy, z.B. eine der folgenden Gruppen:
-CH₃, -C₂H₅, -C₃H₇, -C₄H₉, -C₅H₁₁, -C₆H₁₃, -C₇H₁₅, -C₈H₁₇, -C₉H₁₉, -C₁₀H₂₁, -C₁₁H₂₃, -C₁₂H₂₅, -C₁₃H₂₇, -C₁₄H₂₉, -C₁₅H₃₁, -OCH₃, -OC₂H₅, OC₃H₇, OC₄H₉, OC₅H₁₁, OC₆H₁₃, OC₈H₁₇, OC₉H₁₉, OC₁₀H₂₁, OC₁₃H₂₇, OC₁₄H₂₉, -O-C₁₅H₃₁, 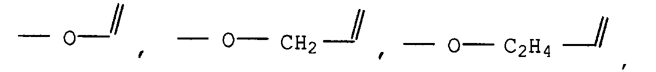
sowie die Zwischenglieder bis 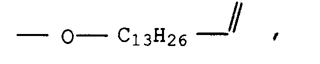 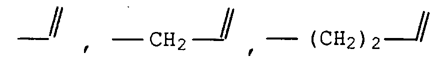
sowie die Zwischenglieder bis 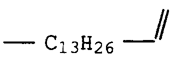 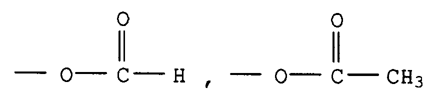
sowie die Zwischenglieder bis 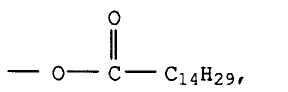 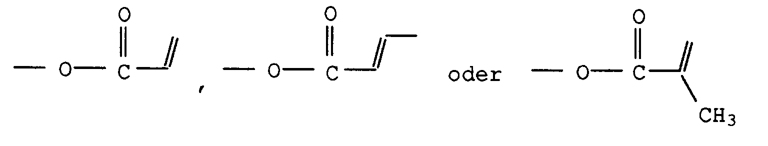
Besonders bevorzugt für Y¹ sind die obigen Alkyl- und Alkoxyreste sowie die Reste der Formeln: 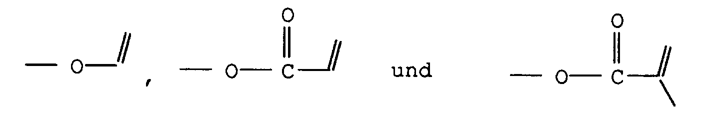
n ist vorzugsweise null oder eins wobei 0 besonders bevorzugt ist.

Die Herstellung der Verbindungen der Formel I ist nach an sich bekannten Methoden möglich.

Einzelheiten zur Herstellung können den Beispielen entnommen werden. Die Verbindungen der Formel I sind zum Teil flüssigkristallin und können in Abhängigkeit von der Struktur chiral smektische oder chiral nematische Phasen ausbilden. Sie sind insbesondere mit anderen flüssigkristallinen, kalamitischen Verbindungen mischbar. Die Verbindungen der Formel I sowie diese enthaltende Mischungen sind für alle Zwecke geeignet, bei denen man üblicherweise flüssigkristalline Verbindungen bzw. Mischungen verwendet.

Zur Einstellung gewünschter Eigenschaften von Mischungen kann es zweckmäßig sein mehr als zwei Verbindungen der Formel I oder Mischungen mit anderen polymerisierbaren Flüssigkristallen zu verwenden, wobei diese Mischungen in situ oder durch mechanisches Mischen hergestellt werden können. Das Anpassen der Phasenzustandsbereiche ist insbesondere auch durch Zusatz von nicht flüssigkristallinen polymerisierbaren Komponenten, sogenannte Reaktivverdünner, wie z.B. Hexandioldiacrylat oder Bisphenol-A-diacrylat, welche hier nur beispielhaft genannt sein sollen, möglich. Die erfindungsgemäßen Verbindungen eignen sich insbesondere als Orientierungsschichten für flüssigkristalline Materialien, als photovernetzbare Kleber, als Monomere zur Herstellung flüssigkristalliner Netzwerke, als Basismaterial zur Herstellung von chiral dotierbaren polymerisierbaren Flüssigkristallsystemen, als polymerisierbare oder flüssigkristalline Materialien für optische Bauelemente, wie Polarisatoren, Verzögerungsplatten oder Linsen.

Weiterhin lassen sich die erfindungsgemäßen Verbindungen allein oder in Mischungen mit anderen flüssigkristallinen oder nicht flüssigkristallinen Verbindungen als Bestandteil von cholesterisch flüssigkristallinen Beschichtungsmitteln verwenden. Solche Beschichtungsmittel können z.B. Lacke, Druckfarben oder Dispersionsfarben sein. Solche chlolesterisch flüssigkristallinen Beschichtungen, die gewünschtenfalls auch polymerisierbare Systeme enthalten, zeigen interessante Farbeffekte mit blickwinkelabhängigem Farbeindruck.

### Beispiele

Im folgenden seien einige, in den Beispielen durchgängig benutzte Abkürzungen aufgeführt:
- K: kristalline Phase
- Ch: cholesterische Phase
- Sₐ: smektische A Phase
- I: isotrope Phase
Die Phasenumwandlungstemperaturen wurden polarisationsmikroskopisch aufgenommen. Die Temperaturkontrolle erfolgte in einem Mettler Mikroskopheiztisch FP80/82.
PE = Petrolether (60 bis 90°C)
EE = Essigsäureethylester
Tol = Toluol 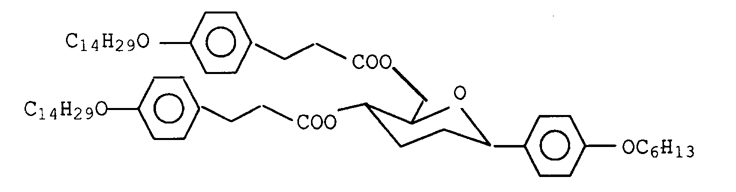
Die Vorstufen zu den Beispielen 1 bis 12 werden nach den folgenden Vorschriften hergestellt:
Als Ausgangsstoff wird jeweils die Verbindung der Formel 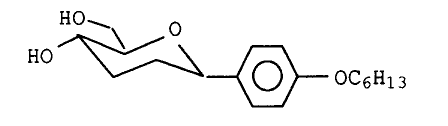
= 1'-(2,3-Didesoxy-β-D-erythro-glucopyranosyl)-4'-hexoxybenzol im folgenden mit (1) bezeichnet, verwendet.

### Vorstufe zu Beispiel 1

100 mg (0,32 mmol) 1'-(2,3-Didesoxy-β-D-erythro-hexopyranosyl)-4'-hexoxybenzol (1) werden in 5 ml Pyridin gelöst und mit 100 mg (0.52 mmol) p-Toluolsulfonylchlorid in 5 ml Dichlormethan versetzt. Nach zwei Stunden werden 2 ml Wasser zugesetzt, nach 30 min. Stehen bei 0°C die Phasen getrennt, die organische Phase mit gesättigter Natriumhydrogencarbonatlösung gewaschen, über Magnesiumsulfat getrocknet und das Lösungsmittel im Vakuum entfernt. Das Rohprodukt wird säulenchromatographisch gereinigt. Auswaage: 68 mg (46 %).

Das 1'-(2,3-Didesoxy-6-toluolsulfonyl-β-D-erythro-hexoxybenzol (68 mg, 0,15 mmol) wird in 15 ml Tetrahydrofuran gelöst auf 0°C gekühlt, mit 6 mg (0,15 mmol) Lithiumaluminiumhydrid versetzt und nach Erwärmung auf Raumtemperatur 8 Stunden unter Rückfluß erhitzt. Nach Abkühlung und Zerstörung überschüssigen Reagenzes mit Ethylacetat wird durch Celite® filtriert, das Lösungsmittel im Vakuum verdampft und das Produkt säulenchromatographisch gereinigt. Auswaage: 26 mg (60 %).

Das Produkt hat die Formel (2)

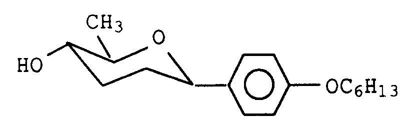

### Vorstufe zu Beispiel 2

### 1'-(6-Fluor-2,3,6-tridesoxy-β-D-erythro-hexopyranosyl)-4'-hexoxybenzol (3)

Eine Lösung von (1) (255 mg, 0,83 mmol) in CH₂Cl₂ (1 ml, trocken) wird zu einer gekühlten (-10°C) und gerührten Lösung von Diethylaminoschwefeltrifluorid (0,4 ml, 3,31 mmol) in CH₂Cl₂ (1 ml) gegeben. Die Temperatur wird innerhalb einer Stunde langsam auf Raumtemperatur gebracht. Das Reaktionsgemisch wird auf Eis gegeben und die wäßrige Phase dreimal mit 5 ml CH₂Cl₂ ausgeschüttelt. Die vereinigten organischen Phasen werden unter reduziertem Druck eingeengt. Der erhaltene Sirup wird säulenchromatographisch aufgearbeitet (Eluent PE: EE 4:1). Das Produkt wurde in einer Ausbeute von 7,4 mg (3 %) erhalten.
R_{f}-Wert: 0,36 (in Tol: EE 1:1).

### Das Produkt hat die Formel (3)

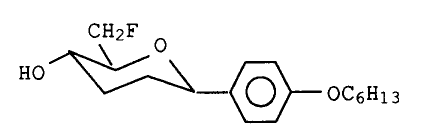

### Vorstufe zu Beispiel 3

### 1'-(6-Chlor-2,3,6-tridesoxy-β-D-erythro-hexopyranosyl)4'-hexoxybenzol

(1) (50,3 mg, 0,16 mmol) wird in DMF (2 ml) gelöst und mit Mesylchlorid (60 mg, 0,52 mmol) versetzt. Danach wird das ganze auf 80°C erwärmt. Nach einer Stunde wird das Lösungsmittel abgezogen. Der Rückstand wird in Methanol (5 ml) aufgenommen und mit einer Spatelspitze Natriummethanolat versetzt. Der Ansatz wird 3 Stunden bei Raumtemperatur gerührt. Nach der Zugabe von saurem Ionenaustauscher wird eine weitere halbe Stunde gerührt. Nach dem Abfiltrieren wird das Lösungsmittel bei reduziertem Druck abgezogen. Der erhaltene Sirup wird säulenchromatographisch aufgearbeitet (Eluent Tol: EE 4:1). Das Produkt (4) wurde in einer Ausbeute von 34,4 mg (64 %) erhalten.
R_{f}-Wert: 0,52 (in Tol: EE 1:1).

Die Verbindung (4) trägt anstelle des F in Formel (3) ein Cl.

### Vorstufe zu Beispiel 4

### 1'-(6-Brom-2,3,6-tridesoxy-β-D-erythro-hexopyranosyl)-4'-hexoxybenzol (5)

Zu einer gekühlten Lösung (0°C) von (1) (50,3 mg, 0,16 mmol) und NBS (58,1 mg, 0,33 mmol) in DMF (2 ml) gibt man über einen Zeitraum von 5 min. eine Lösung von Triphenylphosphin (85,6 mg, 0,33 mmol) in DMF (1 ml). Nach der Zugabe wird der Ansatz zwei Stunden auf 50°C erhitzt. Das Lösungsmittel wird bei reduziertem Druck abgezogen, und der erhaltene Sirup wird säulenchromatographisch aufgearbeitet (Eluent PE: EE 4:1). Das Produkt (5) wurde in einer Ausbeute von 29,3 mg (48 %) erhalten.
R_{f}-Wert: 0,38 (in Tol: EE 1:1)
[α]_{D}²⁰ = + 36° (c= 1,06 in CH₂Cl₂)
Die Verbindung (5) trägt anstelle des F in Formel (3) ein Br.

### Vorstufe zu Beispiel 5

### 1'-(6-Iod-2,3,6-tridesoxy-β-D-erythro-hexopyranosyl)-4'-hexoxybenzol (6)

Eine Mischung von (1) (30,8 mg, 0,1 mmol), Triphenylphosphin (39,3 mg, 0,15 mmol), Imidazol (20,4 mg, 0,3 mmol) und Iod (35,5 mg, 0,14 mmol) in Toluol (2 ml, abs.) werden bei 70°C für 1/2 h gut gerührt. Die Reaktionsmischung wird gekühlt, ein gleicher Teil gesättigter Natriumhydrogencarbonatlösung dazu gegeben, und für 5 Minuten gerührt. Dann gibt man solange Iod zu, bis die Toluol-Phase iodfarben bleibt. Man rührt nochmal 10 Minuten. Der Überschuß an Iod wird durch wäßrige Thiosulfat-Lösung entfernt. Nach der Überführung der Reaktionslösung in einen Scheidetrichter wird die organische Phase mit Toluol verdünnt. Nach der Abtrennung wird die organische Phase mit Wasser extrahiert, über Magnesiumsulfat getrocknet, filtriert und aufkonzentriert. Der erhaltene Rückstand wird säulenchromatographisch aufgearbeitet (Eluent PE: EE 4:1). Das Produkt (6) wurde in einer Ausbeute von 22,3 mg (53 %) erhalten.
R_{f}-Wert: 0,47 (in Tol: EE 1:1)
Die Verbindung (6) trägt anstelle des F in Formel (3) ein J.

### Vorstufe zu Beispiel 6

### 1'-(6-O-tert.-Butyldimethylsilyl-2,3-didesoxy-β-D-erythro-hexopyranosyl)-4'-hexoxybenzol (7)

Zu einer Lösung von (1) (300 mg, 0,97 mmol) in Pyridin (4 ml) gibt man tert.-Butyldimethylsilylchlorid (166 mg, 1,10 mmol). Man läßt den Ansatz für 3 h bei Raumtemperatur stehen. Das Lösungsmittel wird unter reduziertem Druck abgezogen. Um anhaftendes Pyridin zu vertreiben, wird dreimal Toluol abgezogen. Der Rückstand wird in CH₂Cl₂ aufgenommen, über Magnesiumsulfat getrocknet, filtriert und eingeengt. Man erhält die Verbindung (7) in einer Ausbeute von 37,8 mg (92 %).
R_{f}-Wert: 0,62 (in Tol: EE 2:1)
Die Verbindung (7) trägt anstelle des F in Formel (3) eine Gruppe der Formel 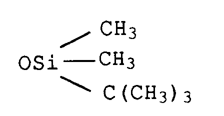
Allgemeine Arbeitsvorschrift zur Herstellung der Verbindungen der Beispiele 1 bis 12:
Eine Lösung der Carbonsäure R¹-OH (1 Teil), von N,N-Dicyclohexylcarbodiimid (1,1 Teile), des Alkohols [Verbindungen (1) bis (7)] (1,1 Teile) und 4-Dimethylaminopyridin (1 Teil) in Dichlormethan wird bei Raumtemperatur stehen gelassen, bis die Veresterung vollständig ist (ca. 2-3 Tage).
Das Lösungsmittel wird im Vakuum abgezogen und der Rückstand säulenchromatographisch mit einem Petrolether/Ethylacetat-Gemisch aufgearbeitet.

### Beispiel 1

### 1'-(4-O-p-octyloxybenzoyl-2,3,6-tridesoxy-β-D-erythro-hexopyranosyl)-4'-hexoxybenzol

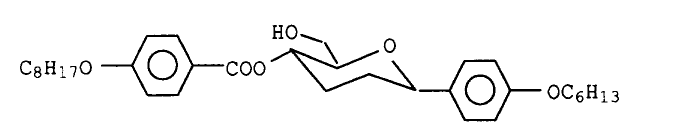

Es wurden analog der oben angegebenen Vorschrift 0,018 mmol Octyloxybenzoesäure und 0,02 mmol der Verbindung (1) umgesetzt. Als Eluent wurde ein Gemisch aus PE:EE 20:1 verwendet.

Das Produkt wurde in einer Ausbeute von 5 mg (40 %) erhalten.
R_{f}-Wert: 0,85 (in Tol:EE 8:1)
[α]_{D}²⁰ = +35°(c= 0,28 in CH₂Cl₂)
Phasenverhalten: K 45.4 Ch 86.6 I

### Beispiel 2

### 1'-(6-Fluor-4-O-p-octyloxybenzoyl-2,3,6-tridesoxy-β-D-erythro-hexopyranosyl)-4'-hexoxybenzol

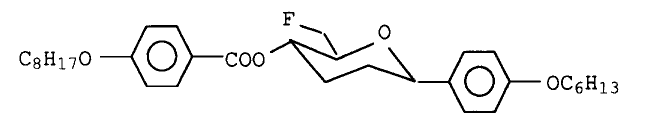

Es wurden analog Beispiel 1 0,018 mmol Octyloxybenzoesäure und 0,02 mmol der Verbindung (3) eingesetzt. Als Eluent wurde ein Gemisch PE:EE 9:1 verwendet.

Das Produkt wurde in einer Ausbeute von 1,5 mg (12 %) erhalten.
R_{f}-Wert: 0,86 (in Tol:EE 8:1)
Die Verbindung weist zwischen 51°C und 64,1°C eine grün/blaue Selektivreflexion auf.
Phasenverhalten: K 51.0 Ch 64,1 I

### Beispiel 3

### 1'-(6-Chlor-4-O-p-octyloxybenzoyl-2,3,6-tridesoxy-β-D-erythrohexopyranosyl)-4'-hexoxybenzol

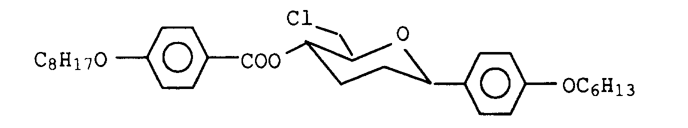

Analog Beispiel 1 wurden 0,1 mmol Octyloxybenzoesäure und 0,11 mmol der Verbindung (4) umgesetzt. Als Eluent diente ein Gemisch PE:EE 11:1.

Das Produkt wurde in einer Ausbeute von 19,3 mg (33 %) erhalten. R_{f}-Wert: 0,89 (in Tol:EE 8:1)
[α]_{D}²⁰ = +27° (c = 1,17 in CH₂Cl₂)
Die Verbindung weist unterhalb 38,5°C beim Abkühlen eine grün/blaue Selektivreflexion auf.
Phasenverhalten: K 47.5 (Ch 38.8) I

### Beispiel 4

### 1'-(6-Brom-4-O-p-octyloxybenzoyl-2,3,6-tridesoxy-β-D-erythro-hexopyranosyl)-4'-hexoxybenzol

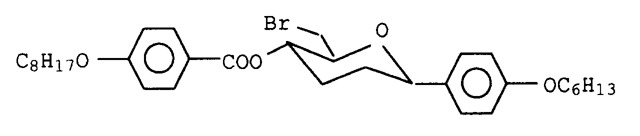

Die Verbindung wurde analog Beispiel 1 hergestellt, es wurden 0,036 mmol Octyloxybenzoesäure und 0,04 mmol Verbindung (5) eingesetzt. Als Eluent wird PE:EE im Verhältnis 20:1 eingesetzt.

Das Produkt wurde in einer Ausbeute von 11,2 mg (47 %) erhalten.
R_{f}-Wert: 0,88 (in Tol:EE 8:1)
[α]_{D}²⁰ = +43° (c = 1,14 in CH₂Cl₂)
Phasenverhalten: K 50.0 (Ch 33.8) I

### Beispiel 5

### 1'-(6-Iod-4-O-p-octyloxybenzoyl-2,3,6-tridesoxy-β-D-erythro-hexopyranosyl)-4'-hexoxybenzol

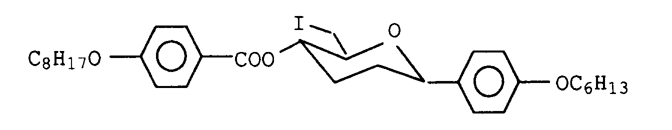

Analog Beispiel 1 wurden 0,045 mmol Octyloxybenzoesäure und 0,05 mmol Verbindung (6) eingesetzt. Als Eluent diente ein Gemisch aus PE:EE im Verhältnis 8:1. Ausbeute: 11 mg (37 %) R_{f}-Wert: 0,91 (in Tol/EE 4:1)
[α]_{D} = 46° (c = 0,91 in CH₂Cl₂)
Phasenverhalten: K 63.9 (Ch 27.3) I

### Beispiel 6

### 1'-(4-O-p-Octyloxybenzoyl-2,3-didesoxy-β-D-erythro-hexopyranosyl)-4'-hexoxybenzol

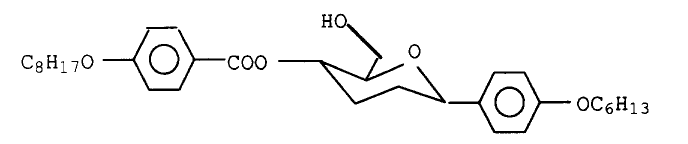

Die Darstellung erfolgt analog Beispiel (1). Es wurden 0,90 mmol der Verbindung 7 und 0,81 mmol Octyloxybenzoesäure eingesetzt. Es wurde ein Produktgemisch des Beispiels 7 und des mit der Butyldimethylsilylgruppe geschützten Beispiels 6 isoliert, welches chromatographisch getrennt wurde. Als Eluent diente PE:EE 8:1.

Zur Entfernung der Butyldimethylsilyl-Schutzgruppe wird zu der Lösung von (28,4 mg, 0,04 mmol) 1'-(6-O-tert.-Butyldimethylsilyl-4-O-p-octyloxybenzoyl-2,3-didesoxy-β-D-erythro-hexopyranosyl)-4'-hexoxybenzol in CH₂Cl₂ (5 ml) Methanol (5 ml) und Amberlite® A-26 (H⁺) zugegeben. Man läßt den Ansatz 3 d bei Raumtemperatur rühren, filtriert den Ionenaustauscher ab und engt die Lösung ein. Der Rückstand wird säulenchromatographisch aufgearbeitet (Eluent PE:EE 8:1).
Das Produkt wurde in einer Ausbeute von 13,4 mg (57 %) erhalten.
R_{f}-Wert: 0,10 (in Tol:EE 6:1)
[α]_{D}²⁰ = +23° (c = 1,10 in CH₂Cl₂)
Phasenverhalten: K 42.0 (Ch 59.0) I

### Beispiel 7

### 1'-(4,6-Di-O-p-Octyloxybenzoyl-2,3-didesoxy-β-D-erythro-hexopyranosyl)-4'-hexoxybenzol

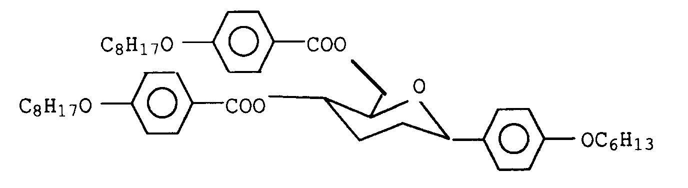

Das Produkt wurde in einer Ausbeute von 201,0 mg (29 %) erhalten.
R_{f}-Wert: 0,52 (in Tol:EE 4:1)
[α]_{D}²⁰ = +52° (c = 1,02 in CH₂Cl₂)
Phasenverhalten: K 59.4 (Ch 32.1) I

### Beispiel 8

### 1'-(4-O-(3-(p-Tetradecyloxyphenyl)propionyl)-2,3-didesoxy-β-D-erythro-hexopyranosyl)-4'-hexoxybenzol

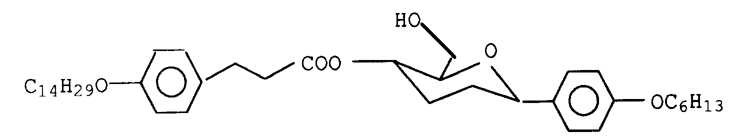

Die Darstellung der Verbindungen der Beispiele 8,9 und 10 erfolgt analog Beispiel 1 durch Umsetzung von 0,4 mmol 3-(p-Tetradecyloxyphenyl)-propionsäure mit 0,22 mmol der Verbindung (1). Die Trennung von Bsp. 8,9,10, erfolgt chormatographisch. Als Eluent diente ein Gemisch aus PE:EE im Verhältnis 12:1

Das Produkt wurde in einer Ausbeute von 51 mg (35 %) erhalten.
R_{f}-Wert: 0,40 (in Tol:EE 4:1)
[α]_{D}²⁰=+20°(c = 0,55 in CH₂Cl₂)
Phasenverhalten: K 84,8 (S_{A} 52.0) I

### Beispiel 9

### 1'-(6-O-(3-(p-Tetradecyloxyphenyl)propionyl)-2,3-didesoxy-β-D-erythro-hexopyranosyl)-4'-hexoxybenzol

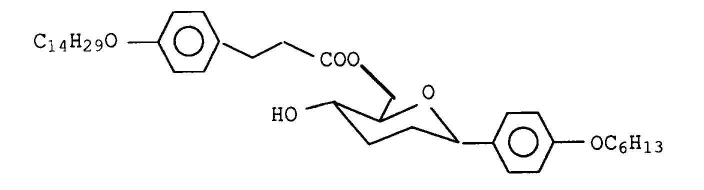

Das Produkt wurde in einer Ausbeute von 78 mg (53 %) erhalten.
R_{f}-Wert: 0,25 (in Tol:EE 4:1)
[α]_{D}²⁰=-7°(c = 1,18 in CH₂Cl₂)
Phasenverhalten: K 68,0 I

### Beispiel 10

### 1'-(4,6-Di-O-(3-(p-Tetradecyloxyphenyl)propionyl)-2,3-didesoxy-β-D-erythro-hexopyranosyl)-4'-hexoxybenzol

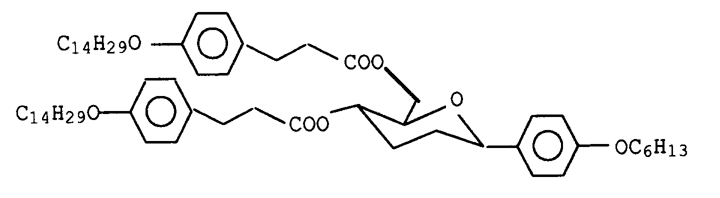

Das Produkt wurde in einer Ausbeute von 5 mg (2 %) erhalten.
R_{f}-Wert: 0,51 (in Tol:EE 4:1)
[α]_{D}²⁰=+20°(c = 0,7 in CH₂Cl₂)
Phasenverhalten: K 72,0 I

### Beispiel 11

### 1'-(4-O-p-Tetradecyloxybenzoyl-6-O-(3-(p-tetradecyloxyphenyl)propionyl)-2,3-didesoxy-β-D-erythro-hexopyranosyl)-4'-hexoxybenzol

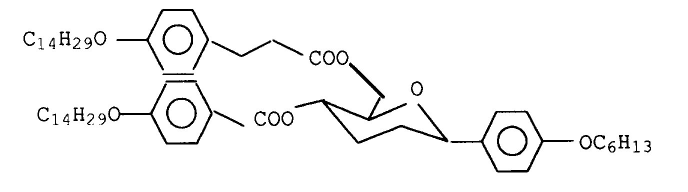

Analog Beispiel 1 wurde Beispiel 11 hergestellt. Eingesetzt wurde 0,4 mmol der Verbindung aus Beispiel 9 und 0,036 mmol p-Tetradecyloxybenzoesäure. Als Eluent wurde PE:EE im Verhältnis 8:1 eingesetzt.

Das Produkt wurde in einer Ausbeute von 6,1 mg (17 %) erhalten.
R_{f}-Wert: 0,94 (in Tol:EE 4:1)
[α]_{D}²⁰=+30°(c = 0,61 in CH₂Cl₂)
Phasenverhalten: K 79,3 (S_{A} 61,2) I

### Beispiel 12

### 1'-(6-O-p-Tetradecyloxybenzoyl-4-O-(3-(p-tetradecyloxyphenyl)propionyl)-2,3-didesoxy-β-D-erythro-hexopyranosyl)-4'-hexoxybenzol

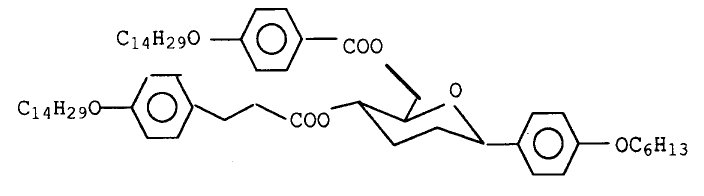

Analog Beispiel 1 wurden 0,009 mmol Tetradecyloxybenzoesäure und 0,01 mmol der Verbindung aus Beispiel 8 eingesetzt. Als Eluent diente ein Gemisch aus PE:EE im Verhältnis 8:1.

Das Produkt wurde in einer Ausbeute von 3,8 mg (58 %) erhalten.
R_{f}-Wert: 0,83 (in Tol:EE 4:1)
Phasenverhalten: K 72,4 I

## Patentansprüche

1. Chirale Verbindungen der allgemeinen Formel I 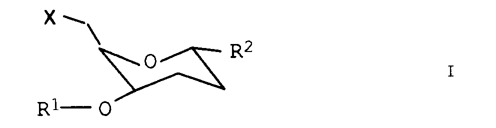 in der
R¹ Wasserstoff oder ein Rest der Formel -Y-Z-Y¹,
R² ein Rest der Formel 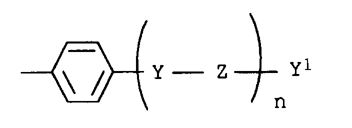
und
X Wasserstoff, Fluor, Chlor, Brom, Jod, Hydroxy oder OR¹ sind, wobei
Y eine direkte Bindung, CO oder C₂- bis C₈-Alkylen, in welchem nichtbenachbarte C-Atome durch O, CO, COO oder OCO ersetzt sein können
Z eine Gruppe, die einen oder mehrere gesättigte oder ungesättigte, gegebenenfalls Heteroatome aufweisende Ringe enthält,
Y¹ C₁- bis C₁₅-Alkyl, C₂- bis C₁₅-Alkenyl, C₁- bis C₁₅-Alkoxy, C₂- bis C₁₅-Alkenyloxy, C₁- bis C₁₅-Alkanoyloxy oder C₃- bis C₁₅-Alkenoyloxy, wobei in diesen Resten nichtbenachbarte Kohlenstoffatome durch Sauerstoff, OCO oder COO ersetzt sein können, und
n 0,1 oder 2 bedeuten.

2. Verbindungen nach Anspruch 1, wobei R¹ ein Rest der Formel Y-Z-Y¹ ist.

3. Verbindungen nach Anspruch 2, wobei Y eine direkte Bindung, CO oder C₁ bis C₈-Alkylen, in welchem nichtbenachbarte C-Atome durch O, CO, COO oder OCO ersetzt sein können, ist.

4. Verbindungen nach Anspruch 2, wobei Z ein gesättigter oder ungesättigter gegebenenfalls Heteroatome enthaltender Ring ist.

5. Verbindungen nach Anspruch 2, wobei Y¹ C₁- bis C₁₅-Alkyl, C₁- bis C₁₅-Alkoxy, C₂- bis C₁₅-Alkenyl, C₂- bis C₁₅-Alkenyloxy, Acryloyloxy oder Methacryloyloxy ist.

6. Verbindungen nach Anspruch 4, wobei Z ein Rest der Formel 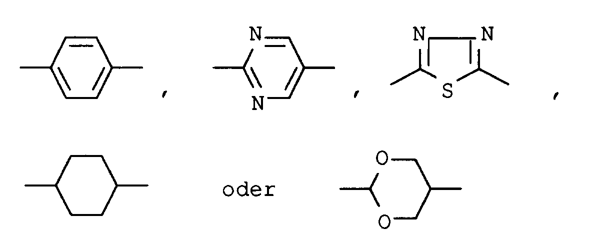 ist.

7. Verbindungen nach Anspruch 2, wobei n 0 oder 1 ist.

8. Verwendung der Verbindungen gemäß den Ansprüchen 1 bis 7 als chirale Komponente in flüssigkristallinen Mischungen für elektrooptische Anwendungen.

9. Verwendung der Verbindungen gemäß den Ansprüchen 1 bis 7 allein oder in Mischungen mit anderen flüssigkristallinen oder nicht flüssigkristallinen Verbindungen in cholesterisch flüssigkristallinen Beschichtungsmitteln.
